(19) Europäisches Patentamt
European Patent Office
Office européen des brevets

(11) Veröffentlichungsnummer: **0 056 866**
**B1**

(12) # EUROPÄISCHE PATENTSCHRIFT

(45) Veröffentlichungstag der Patentschrift: **19.06.85**

(21) Anmeldenummer: **81110719.2**

(22) Anmeldetag: **23.12.81**

(51) Int. Cl.⁴: **C 07 D 271/10**, C 07 D 249/08, C 07 D 233/61, C 07 D 231/12, A 61 K 31/41, A 61 K 31/415, A 61 K 31/495

(54) **Phenylpiperazinderivate von Hetarylphenolen und Hetarylanilinen, ihre Herstellung und diese enthaltende therapeutische Mittel.**

(30) Priorität: **19.01.81 DE 3101502**

(43) Veröffentlichungstag der Anmeldung: **04.08.82 Patentblatt 82/31**

(45) Bekanntmachung des Hinweises auf die Patenterteilung: **19.06.85 Patentblatt 85/25**

(84) Benannte Vertragsstaaten: **AT BE CH DE FR GB IT LI LU NL SE**

(56) Entgegenhaltungen:
**AT - B - 317 203**
**AT - B - 317 209**
**DE - A - 2 335 432**
**DE - A - 2 824 677**
**GB - A - 1 164 572**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Thieme, Peter C., Dr., Dr.-Hans-Hoffmann-Strasse 5, D-6706 Wachenheim (DE)**
Erfinder: **Steiner, Gerd, Dr., Oberer Waldweg 1, D-6719 Kirchheim (DE)**
Erfinder: **Rohr, Wolfgang, Dr., In der Dreispitz 13, D-6706 Wachenheim (DE)**
Erfinder: **Lenke, Dieter, Dr., Kekuleplatz 1, D-6700 Ludwigshafen (DE)**
Erfinder: **Gries, Josef, Dr., Roemerweg 43, D-6706 Wachenheim (DE)**
Erfinder: **Weifenbach, Harald, Dr., Londoner Ring 71, D-6700 Ludwigshafen (DE)**
Erfinder: **Teschendorf, Hans-Juergen, Dr., Rene-Bohn-Strasse 4, D-6700 Ludwigshafen (DE)**
Erfinder: **Hofmann, Hans Peter, Dr., Untere Hart 12, D-6703 Limburgerhof (DE)**
Erfinder: **Kreiskott, Horst, Dr., Am Boehlig, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 056 866 B1

BUNDESDRUCKEREI BERLIN

**Beschreibung**

Die vorliegende Erfindung betrifft neue Phenylpiperazinylpropane von Hetarylphenolen und Hetarylanilinen und ihre physiologisch verträglichen Säureadditionssalze, Verfahren zu ihrer Herstellung und diese Verbindungen enthaltende pharmazeutische Zubereitungen, die vorwiegend sedative, neuroleptische und blutdrucksenkende Eigenschaften aufweisen.

In AT-317 203 und AT-317 209 werden Phenylpiperazinylalkyläther von Imidazolidinonylphenolen als Arzneistoffe mit einem großen Wirkungsspektrum beschrieben, und in der deutschen Patentanmeldung P 3 005 287.0 werden Phenylpiperazinyl-propanole von 1,3,4-Oxadiazolylphenolen vorgeschlagen, die eine blutdrucksenkende Wirkung zeigen. Gegenstand dieser Anmeldung sind modifizierte Derivate, in denen die Seitenkette und teilweise der heterocyclische Ring abgewandelt wurden und die ein unterschiedliches pharmakologisches Wirkungsprofil aufweisen.

Es wurde gefunden, daß Verbindungen der allgemeinen Formel I,

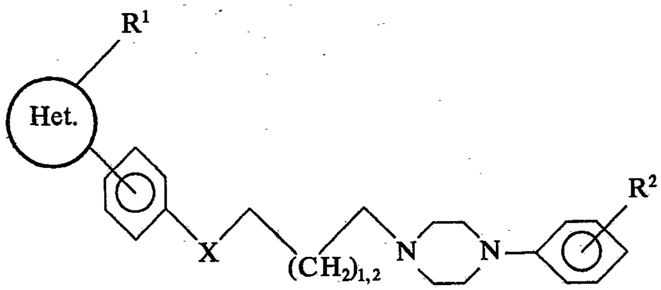

(I)

in denen $R^1$ ein Wasserstoffatom oder einen Alkylrest mit 1 bis 4 C-Atomen, $R^2$ ein Wasserstoffatom, ein Halogenatom, einen Alkylrest mit 1 bis 4 C-Atomen oder einen Alkoxyrest mit 1 bis 3 C-Atomen im Alkyl, wobei $R^2$ als Substituent des Phenylringes ein- oder zweifach vorhanden sein kann, X ein Sauerstoffatom oder eine NH-Gruppe bedeuten und als Heterocyclus Het. ein 1,3,4-Oxadiazolrest, ein Triazolrest, ein Imidazolrest oder ein Pyrazolrest steht, und ihre physiologisch verträglichen Säureadditionssalze wertvolle pharmakologische Eigenschaften aufweisen.

Der Heterocyclus kann sich zu der Ether- bzw. Amino-Gruppe in o-, m- oder p-Stellung befinden.

Als Alkylreste $R^1$ mit 1 bis 4 C-Atomen, geradkettig oder verzweigt, seien Methyl, Äthyl, n-Propyl, Isopropyl, n-Butyl und tert.-Butyl genannt. Die bevorzugten Bedeutungen für $R^1$ sind Wasserstoff und Methyl.

Der Substituent $R^2$ kann in o-, m- oder p-Stellung zum Piperazinsubstituenten im Phenylring des Phenylpiperazins stehen und beispielsweise folgende Bedeutung haben:

Als Halogenatome kommen Fluor, Chlor, Brom und Jod in Betracht, wobei Fluor und Chlor in p- oder m-Stellung bevorzugt sind. Als niedere Alkoxyreste seien der Methoxy-, Ethoxy-, Propoxy- und Isopropoxyrest genannt, wovon Methoxy- und Ethoxy in o-Stellung bevorzugt sind.

Dementsprechend sind als erfindungsgemäße Verbindungen der Formel I beispielsweise zu nennen:

1-[2-(1,3,4-Oxadiazolyl-2)-phenoxy]-3-[4-(4-fluorphenyl)-piperazinyl-1]-propan
1-[2-(1,3,4-Oxadiazolyl-2)-phenoxy]-3-[4-(2-methoxyphenyl)-piperazinyl-1]-propan
1-[2-(5-Methyl-1,3,4-oxadiazolyl-2)-phenoxy]-3-[4-(2-methoxyphenyl)-piperazinyl-1]-propan
1-[2-(5-Methyl-1,3,4-oxadiazolyl-2)-phenoxy]-3-[4-(3-methoxyphenyl)-piperazinyl-1]-propan
1-[2-(1,3,4-Oxadiazolyl-2)-phenoxy]-3-[4-(3-methoxyphenyl)-piperazinyl-1]-propan
1-[2-(1,3,4-Oxadiazolyl-2)-phenoxy]-4-[4-(4-fluorphenyl)-piperazinyl-1]-butan
1-[3-(1,3,4-Oxadiazolyl-2)-phenoxy]-3-[4-(2-methoxyphenyl)-piperazinyl-1]-propan
1-[3-(5-Methyl-1,3,4-oxadiazolyl-2)-phenoxy]-3-[4-(2-methoxyphenyl)-piperazinyl-1]-propan
1-[3-(5-Methyl-1,3,4-oxadiazolyl-2)-phenoxy]-3-[4-(2-methoxyphenyl)-piperazinyl-1]-propan
1-[3-(1,3,4-Oxadiazolyl-2)-phenoxy]-3-[4-(4-fluorphenyl)-piperazinyl-1]-propan
1-[3-(1,3,4-Oxadiazolyl-2)-phenoxy]-3-[4-(3-chlorphenyl)-piperazinyl-1]-propan
1-[3-(5-Methyl-1,3,4-oxadiazolyl-2)-phenoxy]-3-[4-(2-chlorphenyl)-piperazinyl-1]-propan
1-[3-(5-Methyl-1,3,4-oxadiazolyl-2)-phenoxy]-4-[4-(2-methoxyphenyl)-piperazinyl-1]-butan
1-[3-(5-Methyl-1,3,4-oxadiazolyl-2)-phenoxy]-4-[4-(4-fluorphenyl)-piperazinyl-1]-butan
1-[4-(1,2,4-Triazolyl-1)-phenoxy]-3-[4-(4-fluorphenyl)-piperazinyl-1]-propan
1-[4-(Imidazolyl-1)-penoxy]-3-[4-(4-fluorphenyl)-piperazinyl-1]-propan
1-[4-(Pyrazolyl-1)-phenoxy]-3-[4-(4-fluorphenyl)-piperazinyl-1]-propan
1-[4-(1,3,4-Oxadiazolyl-2)-phenoxy]-3-[4-(2-methoxyphenyl)-piperazinyl-1]-propan
1-[4-(1,3,4-Oxadiazolyl-2)-phenoxy]-3-[4-(4-fluorphenyl)-piperazinyl-1]-propan
1-[4-(1,2,4-Triazolyl-1)-phenylamino]-3-[4-(4-fluorphenyl)-piperazinyl-1]-propan
1-[4-(Imidazolyl-1)-phenylamino]-3-[4-(4-fluorphenyl)-piperazinyl-1]-propan

Besonders bevorzugt sind Verbindungen der allgemeinen Formel I nach Anspruch 1, in denen $R^1$ ein

Wasserstoffatom oder Methyl und $R^2$ ein Fluoratom, Chloratom oder eine Methoxygruppe bedeuten, X ein Sauerstoffatom oder eine NH-Gruppe ist und der Heterocyclus Het. sich in m- oder p-Stellung befindet und ihre physiologisch verträglichen Säureadditionssalze.

Die folgenden Verbindungen sind als besonders bevorzugt und wirksam zu nennen:

1-[4-(1,2,4-Triazolyl-1)-phenoxy]-3-[4-(4-fluorphenyl)-piperazinyl-1]-propan
1-[4-(Imidazolyl-1)-phenoxy]-3-[4-(4-fluorphenyl)-piperazinyl-1]-propan
1-[4-(1,2,4-Triazolyl-1)-phenylamino]-3-[4-(4-fluorphenyl)-piperazinyl-1]-propan
1-[4-(Imidazolyl-1)-phenylamino]-3-[4-(4-fluorphenyl)-piperazinyl-1]-propan
1-[4-(1,3,4-Oxadiazolyl-2)-phenoxy]-3-[4-(2-methoxyphenyl)-piperazinyl-1]-propan
1-[4-(1,3,4-Oxadiazolyl-2)-phenoxy]-3-[4-(4-fluorphenyl)-piperazinyl-1]-propan
1-[3-(1,3,4-Oxadiazolyl-2)-phenoxy]-3-[4-(2-methoxyphenyl)-piperazinyl-1]-propan
1-[3-(5-Methyl-1,3,4-oxadiazolyl-2)-phenoxy]-3-[4-(2-methoxyphenyl)-piperazinyl-1]-propan
1-[3-(1,3,4-Oxadiazolyl-2)-phenoxy]-3-[4-(4-fluorphenyl)-piperazinyl-1]-propan
1-[3-(5-Methyl-1,3,4-oxadiazolyl-2)-phenoxy]-4-[4-(2-methoxyphenyl)-piperazinyl-1]-butan

Die erfindungsgemäßen Verbindungen werden hergestellt, indem man ein alkyliertes Hetarylphenol oder Hetarylanilin der allgemeinen Formel II

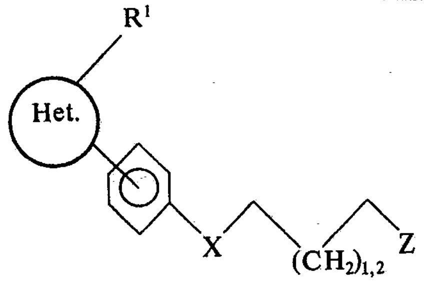

(II)

in der $R^1$ und X die für Formel I angegebenen Bedeutungen haben und in der Z für ein Halogenatom, insbesondere ein Chlor- oder Bromatom, oder für eine sonstige nukleofuge Abgangsgruppe, wie z. B. die Tosyloxy-Gruppe, steht, mit einem Phenylpiperazin der allgemeinen Formel III

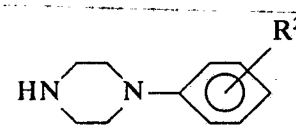

(III)

in der $R^2$ die für Formel I angegebenen Bedeutungen hat, zweckmäßigerweise in einem Lösungsmittel und gegebenenfalls in Gegenwart eines säurebindenden Mittels in an sich bekannter Weise umsetzt und die erhaltenen Verbindungen gegebenenfalls in das Säureadditionssalz einer physiologisch verträglichen Säure überführt.

Die Umsetzungen werden bei Temperaturen von 10 bis 120°C, d. h. bei Raumtemperatur oder bei höheren Temperaturen, zweckmäßig bei Temperaturen von 50 bis 120°C, durchgeführt. Die Umsetzungen können unter Atmosphärendruck oder in einem geschlossenen Gefäß unter erhöhtem Druck, gegebenenfalls unter Erwärmung auf den angegebenen Temperaturbereich, durchgeführt werden.

Zweckmäßigerweise werden die Umsetzungen in Gegenwart eines inerten Verdünnungs- oder Lösungsmittels, beispielsweise eines niederen Alkohols mit 1 bis 4 C-Atomen, wie Methanol, Ethanol oder Propanol, vorzugsweise Isopropanol oder Ethanol, eines niederen gesättigten Dialkylethers, Dialkylglykolethers oder cyclischen Ethers, wie Diethylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Octan, eines niederen aliphatischen Ketons, wie Aceton, Methylethylketon oder Methyl-isobutylketon, eines Dialkylformamids, wie Dimethyl- oder Diethylformamid, von Dimethylsulfoxid oder in Gegenwart von Wasser oder in Mischungen der genannten Lösungsmittel, durchgeführt.

Bevorzugte Lösungsmittel bei der Umsetzung der Verbindungen der Formel II mit einem Phenylpiperazin der Formel III sind niedere Alkohole, insbesondere Ethanol oder Isopropanol, sowie Dimethylsulfoxid, wobei die Umsetzung bevorzugt bei Temperaturen von 50°C bis 120°C und bei Normaldruck durchgeführt wird.

Gegebenenfalls wird die Reaktion in Gegenwart einer katalytischen Menge Natrium- oder Kaliumjodid durchgeführt.

Bei einer zweckmäßigen Ausführungsform zur nukleophilen Substitution des Restes Z wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin oder ein Trialkylamin, wie Trimethylamin oder Triethylamin. Von den Alkaliverbindungen kommen insbesondere die des Natriums und Kaliums in Betracht. Dabei wird die Base in stöchiometrischer Menge oder in geringem Überschuß verwendet. Gegebenenfalls ist es zweckmäßig, das zur Umsetzung verwendete Phenylpiperazinderivat in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich bekannter Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die alkylierten Hetarylphenole bzw. Hetarylaniline der allgemeinen Formel II werden hergestellt, indem man ein Hetarylphenol bzw. Hetarylanilin der allgemeinen Formel IV

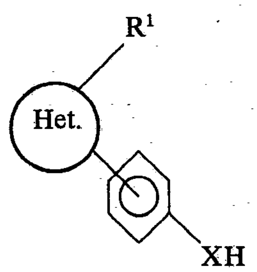

(IV)

in der $R^1$ und X die für Formel I angegebenen Bedeutungen haben, mit einem $\alpha,\omega$-Dihalogen-propan oder -butan, vorzugsweise einem gemischten $\alpha$-Chlor-$\omega$-Brom-Derivat, umsetzt, gemäß folgendem Formelschema:

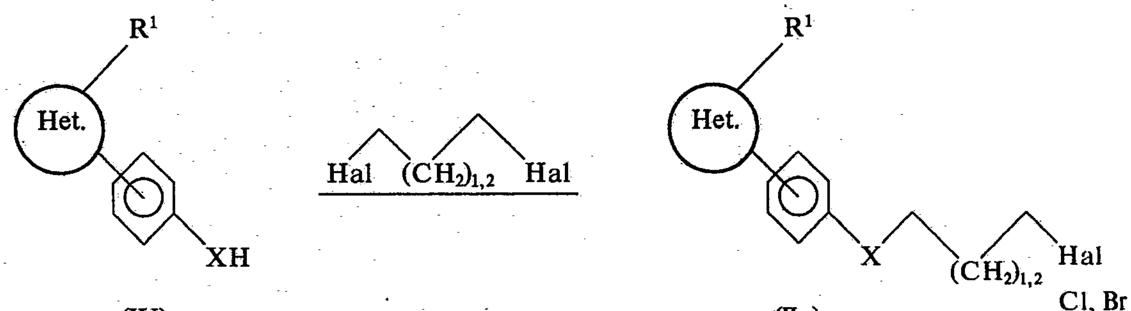

(IV) (IIa)

Die Umsetzungen werden zweckmäßigerweise bei Temperaturen von 0 bis 120° C und unter Normaldruck oder in einem geschlossenen Gefäß unter erhöhten Drücken durchgeführt und sind im allgemeinen innerhalb von 2 bis 15 Stunden beendet. Die Umsetzungen werden zweckmäßigerweise in einem inerten Verdünnungs- oder Lösungsmittel, z. B. einem gesättigten aliphatischen oder cyclischen Ether, wie Dialkyl-Ether, Tetrahydrofuran oder Dioxan, einem Dialkylformamid, wie Dimethylformamid oder Diethylformamid, oder Dimethylsulfoxid durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkoholate, insbesondere Natriumhydrid. Dabei können die Basen im Verhältnis zum eingesetzten Alkylierungsmittel in katalytischer Menge oder in stöchiometrischer Menge bzw. in geringem Überschuß verwendet werden.

Eine weitere Herstellungsmethode für die Verbindungen der Formel I ist die Umsetzung eines Hetarylphenols oder Hetarylanilins der Formel IV mit einem $\omega$-Halogenalkylphenylpiperazin der allgemeinen Formel V,

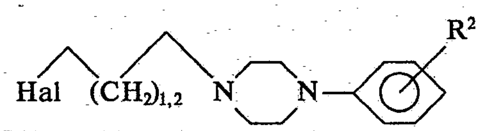

(V)

in der $R^2$ die für Formel I angegebenen Bedeutungen hat und Hal für ein Halogenatom steht.

Die Umsetzungen werden zweckmäßigerweise bei Temperaturen von Raumtemperatur bis 100° C in einem inerten Lösungsmittel, z. B. einem niederen aliphatischen Keton, wie Aceton, Methylethylketon oder Methylisobutylketon, in Gegenwart einer milden Base, vorzugsweise Alkalicarbonat, unter Zusatz einer katalytischen Menge Kaliumjodid oder in einem polaren aprotischen Lösungsmittel, vorzugsweise Dimethylformamid, in Gegenwart einer starken Base, vorzugsweise einem Alkalihydrid, durchgeführt.

Die vollständige Umsetzung hängt von der Reaktionstemperatur ab und ist im allgemeinen innerhalb von 3 bis 40 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallieren aus einem Lösungsmittel, Überführen in eine Säureadditionsverbindung oder durch Säulenchromatographie.

Die $\omega$-Halogenalkyl-phenylpiperazin-Derivate der Formel V sind literaturbekannt und können durch Alkylierung des Phenylpiperazins mit einem $\alpha,\omega$-Dihalogenpropan- bzw. -butan, vorzugsweise einem gemischten Chlor-Brom-Derivat, in Gegenwart einer Base als säurebindendes Mittel in einem inerten Lösungsmittel hergestellt werden, wie in den Beispielen ausgeführt ist.

Die als Ausgangsverbindungen der Formel IV eingesetzten Hetarylphenole bzw. Hetarylaniline sind literaturbekannt (DE-OS 2 811 638, DE-OS 2 803 870, DE-OS 2 510 781; J. med. pharm. Chem. 5, 383—389 [1962]; Bull. Soc. Chim. Fr. [1976], 839—844) und werden auch in der Patentanmeldung P 3 005 287.0 beschrieben.

Gegebenenfalls werden die erhaltenen erfindungsgemäßen Verbindungen in das Säureadditionssalz einer physiologisch verträglichen Säure überführt. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung, Band 10, Seiten 224 bis 225, Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol oder einem niederen Keton, wie Aceton, Methylethylketon oder Methylisobutylketon, oder einem Ether wie Diethylether, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Phenylpiperazinyl-Derivate der allgemeinen Formel (I) durch Auflösen der freien Base der allgemeinen Formel (I) in einer wäßrigen Säurelösung hergestellt werden.

Die erfindungsgemäßen Verbindungen und ihre physiologisch verträglichen Säureadditionssalze können als Pharmaka mit blutdrucksenkender, sedativer und neuroleptischer Wirkung verwendet werden.

Zum Nachweis der pharmakologischen Wirkungen wurden folgende Methoden verwendet:

## Hypotensive Wirkung

Die blutdrucksenkende Wirkung wurde an narkotisierten Ratten (Stamm, Sprague-Dawley, Gewicht: 230 bis 280 g) in Urethan-Narkose (1,78 g/kg intraperitoneal) nachgewiesen. Der Blutdruck wurde in der Arteria carotis gemessen. Die Substanzapplikation erfolgte in eine Vena jugularis (wäßrige Lösung, 1 ml/kg) oder intraperitoneal als Suspension in Traganth (2 ml/kg). Als ED 20% wurden aus der linearen Regression zwischen log Dosis (mg/kg) und relativer Blutdrucksenkung ($\Delta$%) die Dosen ermittelt, welche eine 20%ige Blutdrucksenkung bewirken.

## Sedative Wirkung

4 bis 8 Gruppen von jeweils 3 weiblichen NMRI-Mäusen erhalten die Substanz oral appliziert. Die photoelektrische Bestimmung der durch eine neue Umgebung induzierten Orientierungsmotilität erfolgte 30 min nach der Applikation der Substanzen für eine Dauer von 30 min. Als ED 50% wird die Dosis bestimmt, welche im Vergleich zu Placebo-behandelten Kontrolltieren eine Abnahme der Orientierungshypermotilität um 50% bewirkt.

Einige der beanspruchten Verbindungen wirken außerordentlich stark hypotensiv (Tab. 1). Die Verbindungen Beispiele 1, 3, 6, 7 und 11 senken den Blutdruck narkotisierter Ratten in Dosen, die mit Ausnahme von Verbindung Beispiel 3 deutlich niedriger sind als beim Chlorpromazin. Dagegen ist der Abstand zwischen der als Nebenwirkung zu betrachtenden sedativen Wirkung und der erwünschten hypotensiven Wirkung wesentlich größer als beim Chlorpromazin (Beispiele 1, 3, 6, 7, 11).

Die Verbindungen der Beispiele 10, 14 und 15 (Tab. 2) zeigen als Ausdruck einer sedativ-neuroleptischen Wirkung starke Hemmung der Orientierungshypermotilität von Mäusen. Dabei wird die Wirkung von Chlorpromazin durch Beispiel 10 fast erreicht oder deutlich übertroffen (Beispiele 14 und 15). Die Substanzen wirken weiterhin im Gegensatz zum Chlorpromazin bis zu Dosen von 1 bzw. 10 mg/kg nicht blutdrucksenkend und unterscheiden sich dadurch im günstigen Sinne von Chlorpromazin, bei dem diese für Sedativa oder Neuroleptica unerwünschte Nebenwirkung bereits in niedrigen Dosen beobachtet wird.

Tabelle 1

| Verbindung Beisp. Nr. | Hypotensive Wirkung ED 20%[1]) | R. W.[2]) | Sedative Wirkung ED 50%[3]) | R. W.[2]) | Q[4]) |
|---|---|---|---|---|---|
| 3 | 0,0384 | 0,68 | 20,5 | 0,10 | 534 |
| 1 | 0,008 | 3,25 | 16,4 | 0,13 | 2050 |
| 7 | 0,0120 | 2,17 | 3,74 | 0,56 | 312 |
| 6 | 0,00770 | 3,38 | 3,21 | 0,66 | 417 |
| 11 | 0,0070 | 3,71 | 2,15 | 0,98 | 307 |
| Chlorpromazin | 0,0260 | 1,00 | 2,11 | 1,00 | 81 |

[1]) Dosis, welche den Blutdruck um 20% senkt (Ratte intravenös).
[2]) Relative Wirksamkeit; Chlorpromazin = 1,00.
[3]) Dosis, welche die Motilität um 50% reduziert (Maus per os)

[4]) $Q = \frac{\text{ED 50\% Sedative Wirkung}}{\text{ED 20\% Hypotensive Wirkung}}$

Tabelle 2

| Verbindung Beisp. Nr. | Sedative Wirkung[1]) ED 50% | R. W.[3]) | Hypotensive Wirkung[2]) ED 20% | R. W.[3]) | Q[4]) |
|---|---|---|---|---|---|
| 10 | 3,05 | 0,69 | 1 i.v. | 0,03 | 3,0 |
| 14 | 0,818 | 2,59 | 10 i.p. | 0,02 | 0,08 |
| 15 | 1,62 | 1,3 | 10 i.v. | 0,003 | 0,16 |
| Chlorpromazin | 2,11 | 1,0 | 0,026 i.v.<br>0,164 i.p. | 1,0<br>1,0 | 81,0<br>12,9 |

[1]) Maus, per os.
[2]) Ratte intravenös (i.v.) oder intraperitoneal (i.p.).
[3]) Relative Wirksamkeit; Chlorpromazin = 1,00.

[4]) $Q = \frac{\text{ED 50\% Sedative Wirkung}}{\text{ED 20\% Hypotensive Wirkung}}$

Gegenstand der vorliegenden Erfindung sind demnach auch therapeutische Mittel oder Zubereitungen, die neben üblichen Träger- und Verdünnungsmitteln eine Verbindung der Formel I oder deren physiologisch verträgliches Säureadditionssalz als Wirkstoff enthalten.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/ oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen beispielsweise auch

Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel wie Maisstärke oder Alginsäure, Bindemittel wie Stärke oder Gelatine, Gleitmittel wie Magnesiumstearat oder Talk und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können außerdem Suspendierstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyäthylenglykol bzw. deren Derivaten herstellen.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 100, vorzugsweise 10 bis 80 mg.

Die vorliegende Erfindung wird durch die nachfolgenden Ausführungsbeispiele näher erläutert.

## Beispiel 1

1-[2-(1,3,4-Oxadiazolyl-2)-phenoxy]-3-[4-(2-methoxyphenyl)-piperazinyl-1]-propan · $H_2O$

2,7 g (0,0167 Mol) 2-(1,3,4-Oxadiazolyl-2)-phenol in 80 ml Methylethylketon werden mit 4,5 g (0,0167 Mol) 1-(3-Chlorpropyl)-4-(2-methoxyphenyl)-piperazin, 2,3 g (0,0167 Mol) Kaliumcarbonat und einer Spatelspitze Kaliumjodid versetzt und 20 bis 40 Stunden am Rückfluß gekocht. Nach dem Abkühlen wird vom Rückstand filtriert und die Mutterlauge eingeengt. Der Rückstand wird mit Ether digeriert, abgesaugt und gut mit Ether nachgewaschen. Man erhält nach dem Trocknen 6,2 g (90%) Produkt mit dem Schmp. 132 bis 133° C.

## Beispiel 2

1-[2-(1,3,4-Oxadiazolyl-2)-phenoxy]-3-[4-(4-fluorphenyl)-piperazinyl-1]-propan

Herstellung analog Beispiel 1 unter Einsatz von 1-(3-Chlorpropyl)-4-(4-fluorphenyl)-piperazin. Nach Abdestillieren des Lösungsmittels wird der Rückstand aus Toluol/Petrolether umkristallisiert. Farblose Kristalle (71%) mit Schmp. 102 bis 103° C.

## Beispiel 3

1-[2-(5-Methyl-1,3,4-oxadiazolyl-2)-phenoxy]-3-[4-(2-methoxyphenyl)-piperazinyl-1]-propan · $H_2O$

Herstellung analog Beispiel 1 unter Einsatz von 2-(5-Methyl-1,3,4-oxadiazolyl-2)-phenol. 57% mit Schmp. 99 bis 100° C.

## Beispiel 4

1-[2-(5-Methyl-1,3,4-oxadiazolyl-2)-phenoxy]-3-[4-(2-methoxyphenyl)-piperazinyl-1]-propan · 2 HCl · 1/2 $H_2O$

Herstellung analog Beispiel 1 unter Einsatz von 2-(5-Methyl-1,3,4-oxadiazolyl-2)-phenol und 1-(3-Chlorpropyl)-4-(3-methoxyphenyl)-piperazin. Nach Einengen der Mutterlauge wird der Rückstand in Methylenchlorid/Ether gelöst und mit etherischer Salzsäure das Hydrochlorid gefällt. 56%, Schmp. 147 bis 148° C.

## Beispiel 5

1-[2-(1,3,4-Oxadiazolyl-2)-phenoxy]-3-[4-(3-methoxyphenyl)-piperazinyl-1]-propan · 2,5 HCl · 1/2 $H_2O$

Herstellung analog Beispiel 1 unter Einsatz von 1-(3-Chlorpropyl)-4-(3-methoxyphenyl)-piperazin. Das Rohprodukt wird durch Säulenchromatographie (Kieselgel, Laufmittel Chloroform/Methanol 6/1) gereinigt, in Ether gelöst und mit etherischer Salzsäure in das Hydrochlorid überführt. 56%, Schmp. 129 bis 130° C.

## Beispiel 6

1-[3-(1,3,4-Oxadiazolyl-2)-phenoxy]-3-[4-(2-methoxyphenyl)-piperazinyl-1]-propan · 2,5 HCl · $H_2O$

Herstellung analog Beispiel 1 unter Einsatz von 3-(1,3,4-Oxadiazolyl-2)-phenol. Das Rohprodukt wird in Methylenchlorid/Ether gelöst und mit etherischer Salzsäure in das Hydrochlorid überführt, das anschließend aus Ethanol/Dimethylformamid/Ether umkristallisiert werden kann. 25%, Schmp. 248 bis 249° C.

## Beispiel 7

1-[3-(5-Methyl-1,3,4-oxadiazolyl-2)-phenoxy]-3-[4-(2-methoxyphenyl)-piperazinyl-1]-propan · 2 HCl · 1/2 $H_2O$

Herstellung analog Beispiel 1 unter Einsatz von 3-(5-Methyl-1,3,4-oxadiazolyl-2)-phenol. Das Rohprodukt wird in Methylenchlorid/Ether gelöst und mit etherischer Salzsäure in das Hydrochlorid überführt. 84%, Schmp. 224 bis 225° C.

## Beispiel 8

1-[3-(5-Methyl-1,3,4-oxadiazolyl-2)-phenoxy]-3-[4-(3-methoxyphenyl)-piperazinyl-1]-propan · 2,5 HCl · $H_2O$

Herstellung analog Beispiel 1 unter Einsatz von 3-(5-Methyl-1,3,4-oxadiazolyl-2)-phenol und 1-(3-Chlorpropyl)-4-(3-methoxyphenyl)-piperazin. Das Rohprodukt wird in Methylenchlorid/Ether gelöst und mit etherischer Salzsäure in das Hydrochlorid überführt. Das rohe Hydrochlorid wird in Wasser digeriert, vom unlöslichen Rückstand abfiltriert und das Filtrat alkalisch gestellt. Die gereinigte freie Base wird durch Extraktion mit Methylenchlorid isoliert, in Methylenchlorid/Ether gelöst, filtriert und nochmals mit etherischer Salzsäure in das Hydrochlorid überführt. 28%, Schmp. 148 bis 150° C.

## Beispiel 9

1-[3-(1,3,4-Oxadiazolyl-2)-phenoxy]-3-[4-(4-fluorphenyl)-piperazinyl-1]-propan

Herstellung analog Beispiel 1 unter Einsatz von 3-(1,3,4-Oxadiazolyl-2)-phenol und 1-(3-Chlorpropyl)-4-(4-fluorphenyl)-piperazin. Das Rohprodukt wird aus Toluol/Petrolether umkristallisiert. 68%, Schmp. 105 bis 106° C.

## Beispiel 10

1-[4-(Imidazolyl-1)-phenoxy]-3-[4-(4-fluorphenyl)-piperazinyl-1]-propan

Herstellung analog Beispiel 1 unter Einsatz von 4-(Imidazolyl-1)-phenol und 1-(3-Chlorpropyl)-4-(4-fluorphenyl)-piperazin. Das Rohprodukt wird durch Säulenchromatographie (Kieselgel, Laufmittel Methylenchlorid) gereinigt und aus Ethanol umkristallisiert. 35%, Schmp. 150 bis 151° C.

Beispiel 11

1-[4-(1,3,4-Oxadiazolyl-2)-phenoxy]-3-[4-(2-methoxyphenyl)-piperazinyl-1]-propan

Herstellung analog Beispiel 1 unter Einsatz von 4-(1,3,4-Oxadiazolyl-2)-phenol. Das filtrierte Reaktionsgemisch wird mit ca. 10 ml Aceton versetzt, und die anfallenden Festkörper abgesaugt und mit Ether gewaschen. 69%, Schmp. 139 bis 140° C.

Beispiel 12

1-[4-(1,3,4-Oxadiazolyl-2)-phenoxy]-3-[4-(4-fluorphenyl)-piperazinyl-1]-propan

Herstellung analog Beispiel 1 unter Einsatz von 4-(1,3,4-Oxadiazolyl-2)-phenol und 1-(3-Chlorpropyl)-4-(4-fluorphenyl)-piperazin. Das Rohprodukt wird aus Toluol und anschließend noch aus Ethanol umkristallisiert. 66%, Schmp. 157 bis 158° C.

Beispiel 13

1-[4-(Pyrazolyl-1)-phenoxy]-3-[4-(4-fluorphenyl)-piperazinyl-1]-propan

2,1 g (0,0131 Mol) 4-(Pyrazolyl-1)-phenol in 60 ml abs. Dimethylformamid werden mit 0,66 g (0,0152 Mol) Natriumhydrid (55% in Paraffinöl) versetzt und 1 Stunde bei Raumtemperatur gerührt. Anschließend fügt man 3,99 g (0,0131 Mol) 1-(3-Chlorpropyl)-4-(4-fluorphenyl)-piperazin hinzu und läßt das Reaktionsgemisch 3 Stunden bei 90° C unter Stickstoff als Schutzgas rühren. Nach dem Abkühlen gießt man den Ansatz auf Eiswasser und saugt die ausfallenden Festkörper ab. Das Rohprodukt wird aus Ethanol umkristallisiert. 72%, Schmp. 151 bis 153° C.

Beispiel 14

1-[4-(1,2,4-Triazolyl-1)-phenylamino]-3-[4-(4-fluorphenyl)-piperazinyl-1]-propan · $H_2O$

Herstellung analog Beispiel 13 unter Einsatz von 4-(1,2,4-Triazolyl-1)-phenol. Nach dem Abkühlen des Reaktionsansatzes destilliert man das Lösungsmittel im Ölpumpenvakuum ab, digeriert den Rückstand in Methylenchlorid und reinigt die Lösung nach Abfiltrieren der unlöslichen Rückstände durch Säulenchromatographie (Kieselgel, Methylenchlorid/Methanol 98/2 als Laufmittel). Man kann das so gereinigte Produkt noch aus Ethanol umkristallisieren. 35%, Schmp. 145 bis 146° C.

Beispiel 15

1-[4-(Imidazolyl-1)-phenylamino]-3-[4-(4-fluorphenyl)-piperazinyl-1]-propan

Herstellung analog Beispiel 13 unter Einsatz von 4-(Imidazolyl-1)-phenol. Aufarbeitung analog Beispiel 14. Nach der Säulenchromatographie wird das Produkt aus Ethanol unter Zusatz von Aktivkohle umkristallisiert. 21%, Schmp. 164 bis 166° C.

Beispiel 16

Herstellung von 1-(3-Chlorpropyl)-4-(4-fluorphenyl)-piperazin

30,0 g (0,166 Mol) 1-(4-Fluorphenyl)-piperazin in 50 ml Tetrahydrofuran wird mit 26,1 g (0,166 Mol) 1-Brom-3-chlorpropan und 33,6 g (0,333 Mol) Triethylamin versetzt. Man läßt das Reaktionsgemisch 16 Stunden bei 60 bis 65° C rühren, filtriert nach dem Abkühlen, zieht das Lösungsmittel im Vakuum ab und destilliert das Rohprodukt im Ölpumpenvakuum. Das Produkt destilliert bei 135 bis 145° C/0,05 Torr in 30,0 g (70%) Ausbeute.

Beispiel 17

Analog Beispiel 16 werden die übrigen 1-(3-Chlorpropyl)-piperazine hergestellt: 1-(3-Chlorpropyl)-4-(2-methoxyphenyl)-piperazin und 1-(3-Chlorpropyl)-4-(3-methoxyphenyl)-piperazin.

Beispiel 18

1-[3-(1,3,4-Oxadiazolyl-2)-phenoxy]-3-[4-(3-chlorphenyl)-piperazinyl-1]-propan · 2 HCl · 1,5 $H_2O$

a) 4,5 g (0,0188 Mol) 1-[3-(1,3,4-Oxadiazolyl-2)-phenoxy]-3-chlor-propan in 100 ml Isopropanol werden mit 7,4 g (0,0377 Mol) 1-(3-Chlorphenyl)-piperazin versetzt und 26 Stunden am Rückfluß gekocht. Nach dem Abkühlen dekantiert man die Isopropanol-Phase vom sich abgeschiedenen Öl ab und zieht das Lösungsmittel ab. Der Rückstand wird in Methylenchlorid gelöst, mit Wasser gewaschen, getrocknet und wieder eingeengt. Das Rohprodukt löst man anschließend in Methylenchlorid/Ether und fällt das Hydrochlorid mit etherischer Salzsäure, das dann noch aus Isopropanol/Dimethylformamid/Ether umkristallisiert wird. 12%, Schmp. 209 bis 213°C.

b) Das Vorprodukt 1-[3-(1,3,4-Oxadiazolyl-2)-phenoxy]-3-chlor-propan wird auf folgende Weise hergestellt:
6,6 g (0,152 Mol) Natriumhydrid (55% in Paraffinöl) werden in 130 ml abs. Dimethylsulfoxid vorgelegt. Man läßt dann unter Rühren 24,4 g (0,152 Mol) 3-(1,3,4-Oxadiazolyl-2)-phenol gelöst in 100 ml abs. Dimethylsulfoxid bei Raumtemperatur und anschließend 59,6 g (0,379 Mol) 1-Brom-3-chlorpropan zutropfen. Man läßt dann den Ansatz 12 Stunden bei 50 bis 60°C nachrühren. Zur Aufarbeitung gibt man 500 ml Wasser hinzu und extrahiert dreimal mit je 150 ml Methylenchlorid. Nach Waschen der organischen Phase mit Natriumcarbonat-Lösung, Trocknen und Einengen isoliert man das Rohprodukt, das durch Aufnehmen in Methanol vom Paraffinöl befreit wird und anschließend zur weiteren Reinigung in 4 n Natronlauge durchgeschüttelt wird. Nach Abdekantieren der Lauge nimmt man das Öl mit 2 n Salzsäure auf und extrahiert das Produkt mit Ether. Das Produkt ist auf diese Weise für die weitere Umsetzung genügend rein. Ausbeute: 55%.

Beispiel 19

1-[3-(5-Methyl-1,3,4-oxadiazolyl-2)-phenoxy]-3-[4-(2-chlorphenyl)-piperazinyl-1]-propan · 2,5 HCl · 1/2 $H_2O$

a) Herstellung analog Beispiel 18a unter Einsatz von 1-[3-(5-Methyl-1,3,4-oxadiazolyl-2)-phenoxy]-3-chlor-propan und 1-(2-Chlorphenyl)-piperazin unter Verlängerung des Rückflußkochens auf 60 Stunden. Nach Entfernen des Lösungsmittels im Vakuum reinigt man das Rohprodukt durch Säulenchromatographie (Kieselgel, Laufmittel Chloroform/Essigester 1/1). Das so gereinigte Produkt wird in Ether gelöst, filtriert und mit etherischer Salzsäure in das Hydrochlorid überführt. 26%, Schmp. 205 bis 207°C.

b) Das Vorprodukt 1-[3-(5-Methyl-1,3,4-oxadiazolyl-2)-phenoxy]-3-chlor-propan wird analog Beispiel 18b hergestellt unter Einsatz von 3-(5-Methyl-1,3,4-oxadiazolyl-2)-phenol. Ausbeute: 59%.

Beispiel 20

1-[4-(1,2,4-Triazolyl-1)-phenoxy]-3-[4-(4-fluorphenyl)-piperazinyl-1]-propan

a) 3,0 (0,013 Mol) 1-[4-(1,2,4-Triazolyl-1)-phenoxy]-3-chlor-propan in 50 ml Ethanol werden mit 4,7 g (0,026 Mol) 1-(4-Fluorphenyl)-piperazin versetzt und 35 Stunden am Rückfluß gekocht. Nach dem Abkühlen saugt man die ausgefallenen Festkörper ab. 49%, Schmp. 134 bis 135°C.

b) Das Vorprodukt 1-[4-(1,2,4-Triazolyl-1)-phenoxy]-3-chlor-propan wird analog Beispiel 18b hergestellt unter Einsatz von 4-(1,2,4-Triazolyl-1)-phenol und unter Abänderung der Reaktionsbedingungen auf 3 Stunden Rühren bei Raumtemperatur. Anschließend gießt man den Ansatz auf Eis/Wasser, gibt Kochsalz hinzu und extrahiert mit Ether. Nach Trocknen und Einengen der organischen Phase erhält man das Rohprodukt, das durch Aufnehmen in Methanol vom Paraffinöl befreit wird. Nach Umkristallisieren aus Cyclohexan erhält man 48% Produkt mit Schmp. 81 bis 82°C.

Beispiel 21

1-[2-(1,3,4-Oxadiazolyl-2)-phenoxy]-4-[4-(4-fluorphenyl)-piperazinyl-1]-butan · 3 HCl · $H_2O$

a) 4,9 g (0,194 Mol) 1-[2-(1,3,4-Oxadiazolyl-2)-phenoxy]-4-chlor-butan in 50 ml Dimethylsulfoxid werden mit 3,5 g (0,0194 Mol) 1-(4-Fluorphenyl)-piperazin versetzt und 30 Stunden bei 80°C gerührt. Anschließend gießt man den Ansatz in Eis/Wasser, säuert mit verd. Salzsäure an und extrahiert mit Ether. Die wäßrige Phase wird dann mit verd. Natronlauge alkalisch gestellt und wiederum mit Ether extrahiert. Nach Waschen und Trocknen der basischen Etherphase erhält man beim Einengen das Rohprodukt, das zur Reinigung in Ether gelöst und mit etherischer Salzsäure in das

Hydrochlorid überführt wird. Nach Umkristallisieren aus Ethanol erhält man 28% Produkt mit Schmp. 240 bis 241°C.

b) Das Vorprodukt 1-[2-(1,3,4-Oxadiazolyl-2)-phenoxy]-4-chlor-butan wird auf folgende Weise hergestellt:
2,69 g (0,0618 Mol) Natriumhydrid (55% in Paraffinöl) werden in 100 ml abs. Dimethylsulfoxid vorgelegt. Man fügt portionsweise 10,0 g (0,0618 Mol) 2-(1,3,4-Oxadiazolyl-2)-phenol hinzu und läßt dann unter Rühren bei 15°C 39,2 g (0,308 Mol) 1,4-Dichlorbutan zutropfen. Man läßt den Ansatz 20 Stunden bei 50°C nachrühren und gießt nach dem Abkühlen auf Eis/-Wasser. Nach Ansäuern mit verd. Salzsäure trennt man die sich gebildete untere organische Phase ab und extrahiert die wäßrige Phase mit Methylenchlorid. Die vereinigten organischen Phasen werden durch Destillation vom Lösungsmittel und überschüssigen 1,4-Dichlorbutan befreit. Das Rohprodukt wird noch aus Ligroin umkristallisiert, Ausbeute: 37%.

Beispiel 22

1-[3-(5-Methyl-1,3,4-oxadiazolyl-2)-phenoxy]-4-[4-(4-fluorphenyl)-piperazinyl-1]-butan

a) Herstellung analog Beispiel 21a unter Einsatz von 1-[3-(5-Methyl-1,3,4-oxadiazolyl-2)-phenoxy]-4-chlor-butan unter Verkürzung der Reaktionszeit auf 12 Stunden. Nach dem Abkühlen des Reaktionsgemisches und mehrstündigem Stehenlassen bei Raumtemperatur saugt man die ausgefallenen Festkörper ab und wäscht gut mit Ether nach. Das Rohprodukt wird aus Toluol umkristallisiert. 41% mit Schmp. 146 bis 147°C.

b) Das Vorprodukt 1-[3-(5-Methyl-1,3,4-oxadiazolyl-2)-phenoxy]-4-chlor-butan wird analog Beispiel 21b hergestellt. Die abgetrennte untere organische Phase nach der sauren Aufarbeitung wird mit wenig Methylenchlorid versetzt, getrocknet und eingeengt. Der bei 0°C ausgefallene Niederschlag wird abgesaugt und mit Petrolether gewaschen. Nach Umkristallisieren aus Ligroin erhält man 51% Produkt.

Beispiel 23

1-[3-(5-Methyl-1,3,4-oxadiazolyl-2)-phenoxy]-4-[4-(2-methoxyphenyl)-piperazinyl-1]-butan · 2 HCl · 1/2 $H_2O$

a) Herstellung analog Beispiel 21a unter Einsatz von 1-[3-(5-Methyl-1,3,4-oxadiazolyl-2)-phenoxy]-4-chlor-butan und 1-(2-Methoxyphenyl)-piperazin. Nach Überführen in das Hydrochlorid mit etherischer Salzsäure erhält man 84% Produkt mit Schmp. 193 bis 194°C.

b) Die Herstellung des Vorproduktes 1-[3-(5-Methyl-1,3,4-oxadiazolyl-2)-phenoxy]-4-chlor-butan ist in Beispiel 22b beschrieben.

Beispiele für pharmazeutische Zubereitungen

Beispiele für Tabletten

| | | |
|---|---|---|
| 1. | Ein Wirkstoff der Formel I | 10 mg |
| | Lactose | 200 mg |
| | Methylcellulose | 15 mg |
| | Maisstärke | 50 mg |
| | Talkum | 11 mg |
| | Magnesiumstearat | 4 mg |
| 2. | Ein Wirkstoff der Formel I | 20 mg |
| | Lactose | 178 mg |
| | Avicel | 80 mg |
| | Polywachs 6000 | 20 mg |
| | Magnesiumstearat | 2 mg |
| 3. | Ein Wirkstoff der Formel I | 50 mg |
| | Polyvinylpyrrolidon (mittl. M. G. 25 000) | 170 mg |
| | Polyethylenglykol (mittl. M. G. 4000) | 14 mg |
| | Hydroxypropylmethylcellulose | 40 mg |
| | Talkum | 4 mg |
| | Magnesiumstearat | 2 mg |

Zu 3.
Der Wirkstoff wird mit Polyvinylpyrrolidon in 10%iger wäßriger Lösung befeuchtet, durch ein Sieb mit der lichten Maschenweite 1,0 mm getrieben und bei 50° C getrocknet. Dieses Granulat wird mit Polyethylenglykol (mittl. M. G. 4000), Hydroxypropylmethylcellulose, Talkum und Magnesiumstearat vermischt und zu Tabletten à 280 mg verpreßt.

4. Beispiel für Dragees

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50° C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum bestehen.

5. Kapselformulierung

| | |
|---|---|
| Ein Wirkstoff der Formel I | 5,0 mg |
| Magnesiumstearat | 2,0 mg |
| Milchzucker | 19,3 mg |

6. Injektionslösung

| | |
|---|---|
| Ein Wirkstoff der Formel I | 10 mg |
| Natriumchlorid | 9 mg |
| Destilliertes Wasser, q. s. auf 1,0 ml | |

**Patentansprüche**

1. Verbindungen der allgemeinen Formel I,

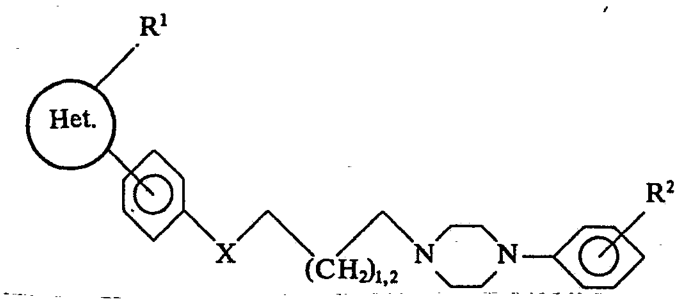

(I)

in denen $R^1$ ein Wasserstoffatom oder einen Alkylrest mit 1 bis 4 C-Atomen, $R^2$ ein Wasserstoffatom, ein Halogenatom, einen Alkylrest mit 1 bis 4 C-Atomen oder einen Alkoxyrest mit 1 bis 3 C-Atomen im Alkyl, wobei $R^2$ als Substituent des Phenylringes ein- oder zweifach vorhanden sein kann, X ein Sauerstoffatom oder eine NH-Gruppe bedeuten und als Heterocyclus Het. ein 1,3,4-Oxadiazolrest, ein Triazolrest, ein Imidazolrest oder ein Pyrazolrest steht, und ihre physiologisch verträglichen Säureadditionssalze.

2. Verbindungen nach Anspruch 1, in denen $R^1$ ein Wasserstoffatom oder Methyl und $R^2$ ein Fluoratom, Chloratom oder eine Methoxygruppe bedeuten, X ein Sauerstoffatom oder eine NH-Gruppe ist und der Heterocyclus Het. sich in m- oder p-Stellung befindet und ihre physiologisch verträglichen Säureadditionssalze.

3. 1-[4-(Imidazolyl-1)-phenoxy]-3-[4-(4-fluorphenyl)-piperazinyl-1]-propan.

4. 1-[4-(1,2,4-Triazolyl-1)-phenylamino]-3-[4-(4-fluorphenyl)-piperazinyl-1]-propan.

5. 1-[4-(Imidazolyl-1)-phenylamino]-3-[4-(4-fluorphenyl)-piperazinyl-1]-propan.

6. 1-[4-(1,3,4-Oxadiazolyl-2)-phenoxy]-3-[4-(2-methoxyphenyl)-piperazinyl-1]-propan.

7. 1-[3-(1,3,4-Oxadiazolyl-2)-phenoxy]-3-[4-(2-methoxyphenyl)-piperazinyl-1]-propan.

8. 1-[3-(5-Methyl-1,3,4-oxadiazolyl-2)-phenoxy]-3-[4-(2-methoxyphenyl)-piperazinyl-1]-propan.

9. 1-[2-(5-Methyl-1,3,4-oxadiazolyl-2)-phenoxy]-3-[4-(2-methoxyphenyl)-piperazinyl-1]-propan.

10. 1-[2-(1,3,4-Oxadiazolyl-2)-phenoxy]-3-[4-(2-methoxyphenyl)-piperazinyl-1]-propan.

11. Verfahren zur Herstellung von Verbindungen der allgemeinen Formel I nach Anspruch 1, dadurch gekennzeichnet, daß man ein alkyliertes Hetarylphenol oder Hetarylanilin der allgemeinen Formel II

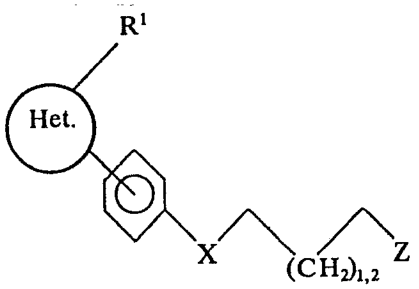

(II)

in der $R^1$ und X die für Formel I angegebenen Bedeutungen haben und in der Z für ein Halogenatom oder für eine nukleofuge Abgangsgruppe steht, mit einem Phenylpiperazin der allgemeinen Formel III

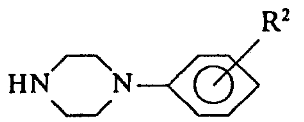

(III)

in der $R^2$ die für Formel I angegebenen Bedeutungen hat, zweckmäßigerweise in einem Lösungsmittel und gegebenenfalls in Gegenwart eines säurebindenden Mittels in an sich bekannter Weise umsetzt und die erhaltenen Verbindungen gegebenenfalls in das Säureadditionssalz einer physiologisch verträglichen Säure überführt.

12. Verfahren zur Herstellung von Verbindungen der allgemeinen Formel I nach Anspruch 1, dadurch gekennzeichnet, daß man ein Hetarylphenol oder Hetarylanilin der allgemeinen Formel IV

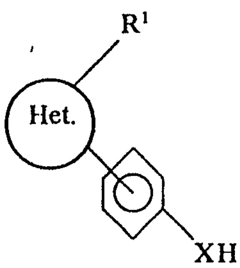

(IV)

in der $R^1$ und X die für Formel I angegebenen Bedeutungen haben, mit einem $\omega$-Halogenalkyl-phenylpiperazin der allgemeinen Formel V,

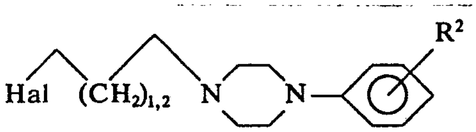

(V)

in der $R^2$ die für Formel I angegebenen Bedeutungen hat und Hal für ein Halogenatom steht, zweckmäßigerweise bei Temperaturen von Raumtemperatur bis 100° C in einem inerten Lösungsmittel in Gegenwart einer milden Base unter Zusatz einer katalytischen Menge Kaliumjodid oder in einem polaren aprotischen Lösungsmittel in Gegenwart einer starken Base in an sich bekannter Weise umsetzt und die erhaltenen Verbindungen gegebenenfalls in das Säureadditionssalz einer physiologisch verträglichen Säure überführt.

13. Therapeutisches Mittel, gekennzeichnet durch einen Gehalt an einer Verbindung der Formel I oder ihres physiologisch verträglichen Säureadditionssalzes als Wirkstoff neben üblichen Trägerstoffen und Verdünnungsmitteln.

## Claims

1. A compound of the general formula I

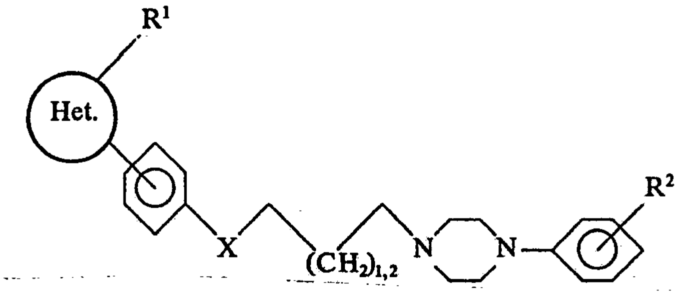

(I)

where $R^1$ is hydrogen or alkyl of 1 to 4 carbon atoms, $R^2$ is hydrogen, halogen, alkyl of 1 to 4 carbon atoms or alkoxy where alkyl is of 1 to 3 carbon atoms, the phenyl ring can be monosubstituted or disubstituted by $R^2$, X is oxygen or an NH group, and the heterocyclic structure Het. is 1,3,4-oxadiazolyl, triazolyl, imidazolyl or pyrazolyl, and its physiologically tolerated addition salts with acids.

2. A compound as claimed in claim 1, where $R^1$ is hydrogen or methyl, $R^2$ is fluorine, chlorine or methoxy, X is oxygen or an NH group, and the heterocyclic structure Het. is located in the m- or p-position, and its physiologically tolerated addition salts with acids.

3. 1-[4-(Imidazol-1-yl)-phenoxy]-3-[4-(4-fluorophenyl-piperazin-1-yl]-propane.

4. 1-[4-(1,2,4-Triazol-1-yl)-phenylamino]-3-[4-(4-fluorophenyl)-piperazin-1-yl]-propane.

5. 1-[4-(Imidazol-1-yl)-phenylamino]-3-[4-(4-fluorophenyl)-piperazin-1-yl]-propane.

6. 1-[4-(1,3,4-Oxadiazol-2-yl)-phenoxy]-3-[4-(2-methoxyphenyl)-piperazin-1-yl]-propane.

7. 1-[3-(1,3,4-Oxadiazol-2-yl)-phenoxy]-3-[4-(2-methoxyphenyl)-piperazin-1-yl]-propane.

8. 1-[3-(5-Methyl-1,3,4-oxadiazol-2-yl)-phenoxy]-3-[4-(2-methoxyphenyl)-piperazin-1-yl]-propane.

9. 1-[2-(5-Methyl-1,3,4-oxadiazol-2-yl)-phenoxy]-3-[4-(2-methoxyphenyl)-piperazin-1-yl]-propane.

10. 1-[2-(1,3,4-Oxadiazol-2-yl)-phenoxy]-3-[4-(2-methoxyphenyl)-piperazin-1-yl]-propane.

11. A process for the preparation of a compound of the general formula I as claimed in claim 1, wherein an alkylated hetarylphenol or hetarylaniline of the general formula II

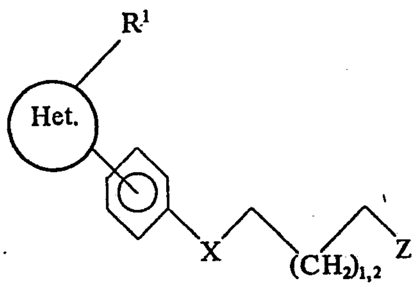

(II)

where $R^1$ and X have the meanings given for formula I and Z is halogen or a nucleofugic leaving group, is reacted in a conventional manner with a phenylpiperazine of the general formula III

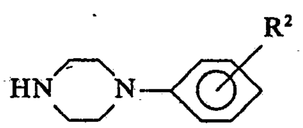

(III)

where $R^2$ has the meanings given for formula I, advantageously in a solvent and in the presence or absence of an acid acceptor, and the resulting compound is converted, if appropriate, into the addition salt of a physiologically tolerated acid.

12. A process for the preparation of a compound of the general formula I as claimed in claim 1, wherein a hetarylphenol or hetarylaniline of the general formula IV

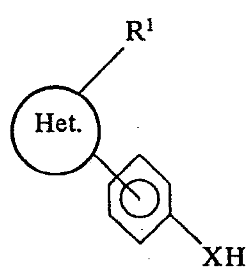

(IV)

where $R^1$ and X have the meanings given for formula I, is reacted in a conventional manner with a phenylpiperazinyl-ω-haloalkane of the general formula V

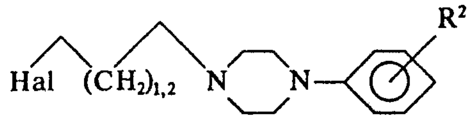

(V)

where $R^2$ has the meanings given for formula I and Hal is halogen, advantageously at from room temperature to 100° C in an inert solvent, in the presence of a mild base, with the addition of a catalytic amount of potassium iodide, or in a polar aprotic solvent in the presence of a strong base, and the resulting compound is converted, if appropriate, into the addition salt of a physiologically tolerated acid.

13. A therapeutic agent which contains, as the active compound, a compound of the formula I or its physiologically tolerated addition salt with an acid, as well as conventional carriers and diluents.

**Revendications**

1. Composés de la formule générale I

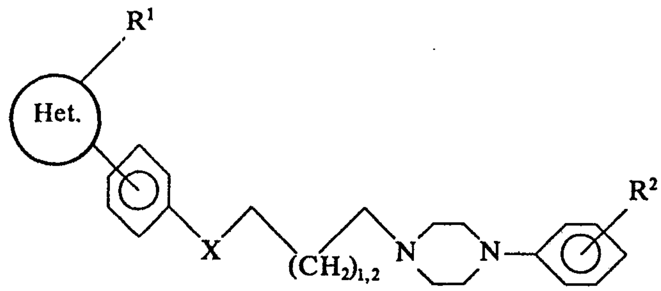

(I)

dans laquelle $R^1$ désigne un atome d'hydrogène ou un radical alkyle en $C_1$ à $C_4$ et $R^2$ un atome d'hydrogène ou d'halogène ou un radical alkyle en $C_1$ à $C_4$ ou un groupe alcoxy à radical alkyle en $C_1$ à $C_3$, le noyau phényle pouvant porter un ou deux substituants $R^2$, X désigne un atome d'oxygène ou un groupe —NH et le groupe hétérocyclique Het étant un groupe 1,3,4-oxadiazole, triazole, imidazole ou pyrazole, ainsi que les sels d'addition d'acides physiologiquement acceptables de ces composés.

2. Composés suivant la revendication 1, pour lesquels $R^1$ est un atome d'hydrogène ou un radical méthyle, $R^2$ un atome de fluor ou de chlore ou un radical méthoxy, X un atome d'oxygène ou un groupe —NH, le groupe hétérocyclique Het se trouvant en position méta ou para, ainsi que leurs sels d'addition d'acides physiologiquement acceptables.

3. Le 1-[4-(imidazolyl-1)-phénoxy]-3-[4-(4-fluoro-phényl)-pipérazinyl-1]-propane.

4. Le 1-[4-(1,2,4-triazolyl-1)-phényl-amino]-3-[4-(4-fluoro-phényl)-pipérazinyl-1]-propane.

5. Le 1-[4-(imidazolyl-1)-phényl-amino]-3-[4-(4-fluoro-phényl)-pipérazinyl-1]-propane.

6. Le 1-[4-(1,3,4-oxadiazolyl-2)-phénoxy]-3-[4-(2-méthoxy-phényl)-pipérazinyl-1]-propane.

7. Le 1-[3-(1,3,4-oxadiazolyl-2)-phénoxy]-3-[4-(2-méthoxy-phényl)-pipérazinyl-1]-propane.

8. Le 1-[3-(5-méthyl-1,3,4-oxadiazolyl-2)-phénoxy]-3-[4-(2-méthoxy-phényl)-pipérazinyl-1]-propane.

9. Le 1-[2-(5-méthyl-1,3,4-oxadiazolyl-2)-phénoxy]-3-[4-(2-méthoxy-phényl)-pipérazinyl-1]-propane.

10. Le 1-[2-(1,3,4-oxadiazolyl-2)-phénoxy]-3-[4-(2-méthoxy-phényl)-pipérazinyl-1]-propane.

11. Procédé de préparation de composés de la formule générale I selon la revendication 1, caractérisé en ce que l'on fait réagir un hétéro-aryl-phénol alkylé ou une hétéro-aryl-aniline alkylée de la formule générale II

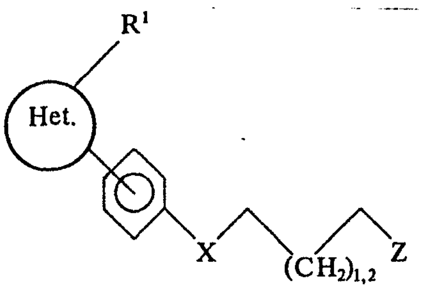

(II)

dans laquelle $R^1$ et X possèdent les significations définies pour la formule I et Z désigne un atome d'halogène ou un groupe nucléofuge clivable, de façon connue en soi, avantageusement dans un solvant et en présence d'un agent fixant les acides, avec une phényl-pipérazine de la formule générale III

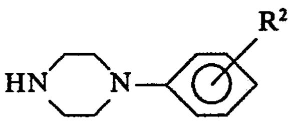

(III)

dans laquelle $R^2$ possède les significations définies pour la formule I, le composé obtenu pouvant ensuite être transformé éventuellement en sel d'addition d'un acide physiologiquement acceptable.

12. Procédé de préparation de composés de la formule générale I selon la revendication 1, caractérisé en ce que l'on fait réagir un hétéro-aryl-phénol ou une hétéro-aryl-aniline de la formule générale IV

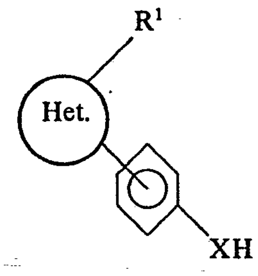

(IV)

dans laquelle $R^1$ et X possèdent les significations définies pour la formule I, de façon connue en soi, entre la température ordinaire et 100° C, soit dans un solvant inerte en présence d'une base faible et d'une quantité catalytique d'iodure de potassium, soit dans un solvant polaire aprotique en présence d'une base forte, avec une $\omega$-halo-alkyl-phényl-pipérazine de la formule générale V

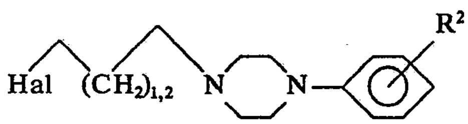

(V)

dans laquelle $R^2$ possède les significations définies pour la formule I et Hal désigne un atome d'halogène, le composé obtenu pouvant éventuellement être transformé en un sel d'addition d'un acide physiologiquement acceptable.

13. Composition pharmaceutique, caractérisée en ce qu'elle contient comme principe actif un composé de la formule I ou un sel d'addition d'un acide physiologiquement acceptable d'un tel composé, ainsi que des matières supports et diluants usuels.